Europäisches Patentamt

European Patent Office

Office européen des brevets

(11) Numéro de publication : **0 381 567 B1**

(19)

# (12) FASCICULE DE BREVET EUROPEEN

(45) Date de publication du fascicule du brevet :
29.09.93 Bulletin 93/39

(51) Int. Cl.⁵ : **G02C 7/06, A61F 2/14, A61F 2/16**

(21) Numéro de dépôt : **90400237.5**

(22) Date de dépôt : **29.01.90**

(54) **Lentille optique à vision simultanée pour la correction de la presbytie.**

(30) Priorité : **03.02.89 FR 8901417**

(43) Date de publication de la demande :
**08.08.90 Bulletin 90/32**

(45) Mention de la délivrance du brevet :
**29.09.93 Bulletin 93/39**

(84) Etats contractants désignés :
**CH DE ES GB IT LI NL SE**

(56) Documents cités :
**EP-A- 0 169 599**
**GB-A- 2 139 375**

(73) Titulaire : **ESSILOR INTERNATIONAL, Cie
Générale d'Optique
1 Rue Thomas Edison, Echat 902
F-94028 Créteil Cédex (FR)**

(72) Inventeur : **Miege, Christian
15 Rue La Fayette
F-95600 Eaubonne (FR)**
Inventeur : **Monteil, Pierre
10 Rue de Lunain
F-75014 Paris (FR)**
Inventeur : **Obrecht, Gérard
20 Rue Emile Zola
F-92130 Issy les Moulineaux (FR)**

(74) Mandataire : **CABINET BONNET-THIRION
95 Boulevard Beaumarchais
F-75003 Paris (FR)**

## Description

La présente invention a d'une manière générale pour objet une lentille optique, pour corriger la presbytie, c'est-à-dire une lentille permettant de compenser un manque d'accommodation du cristallin, l'amplitude d'accommodation diminuant avec l'âge.

Une telle lentille optique doit être en mesure de permettre au presbyte qui en est équipé de voir net un objet situé à n'importe quelle distance (distance éloignée, intermédiaire ou rapprochée).

Cette lentille est du genre dit à vision simultanée progressive suivant lequel la partie centrale dite zone optique, celle-ci ayant un diamètre sensiblement égal à celui de la pupille (à un niveau de luminance basse), comporte des zones annulaires permettant la vision de loin, la vision intermédiaire et la vision de près avec la zone de vision de près à l'intérieur de la zone de vision de loin ou l'inverse.

Il peut s'agir aussi bien d'une lentille de contact, que d'un implant intra-oculaire, ou que d'une lentille intra-cornéenne.

Une lentille optique unifocale est usuellement caractérisée par sa puissance, égale, en dioptries, à l'inverse de sa distance focale, tous les rayons lumineux incidents parallèles à son axe optique passant ensuite par son foyer quelle que soit leur hauteur initiale par rapport à cet axe.

Une lentille optique bifocale comportant deux zones concentriques bien définies possède deux puissances discrètes correspondant, chacune respectivement, l'une à la correction de l'amétropie, en vision de loin, l'autre à la compensation de la presbytie, en vision de près. La différence entre ces deux puissances est appelée addition.

Mais, en toute rigueur, dans le cas d'une lentille à vision simultanée progressive, ces caractéristiques de puissance, qui, pour le porteur, correspondent, du point de vue ophtalmique, à ce dont il a besoin pour disposer d'une vision satisfaisante, se trouvent plus ou moins en défaut, la zone optique utile d'une telle lentille progressive ne possèdant pas à proprement parler de puissances stabilisées.

C'est la raison pour laquelle, il sera utilisé, dans ce qui suit, pour désigner l'inverse de la distance à laquelle un rayon lumineux parallèle à l'axe optique de la lentille et situé à une hauteur $\underline{h}$ par rapport à celui-ci recoupe cet axe après traversée de la lentille, le terme de "proximité" plutôt que celui de "puissance".

En pratique, la proximité d'une lentille optique destinée à un presbyte doit présenter au moins deux plages de valeurs distinctes suivant les besoins visuels du moment de celui-ci, à savoir une plage de valeurs plus particulièrement destinée à sa vision de loin et une plage de valeurs plus particulièrement destinée à sa vision de près, cette dernière se déduisant de la précédente par l'ajout d'une "addition" déterminée, avec, en continu entre ces valeurs, des valeurs intermédiaires de raccordement, l'ensemble répondant à une quelconque loi d'évolution à déterminer.

Le problème, en l'espèce, est que, par rapport à la solution qui semblerait théoriquement idéale impliquant l'obtention d'une évolution rapide entre la valeur de la proximité pour la vision de loin et sa valeur pour la vision de près, il n'existe pas de loi d'évolution susceptible de conduire de manière universelle à de bons résultats pour tous, la meilleure loi d'évolution pour chacun dépendant notamment de l'addition qui lui est nécessaire.

Une loi d'addition linéaire ou quasi linéaire par exemple, ou une loi unique quelle que soit l'addition, ne conduisent en pratique pas à de bons résultats, le porteur ne voyant alors réellement bien ni de loin ni de près.

La présente invention est fondée sur la découverte, non mise en évidence jusqu'à ce jour, que, en faisant en sorte que la courbe représentative de la proximité en fonction de la hauteur par rapport à l'axe se situe dans certaines limites caractéristiques de l'addition nécessaire au porteur, il était possible, au contraire, d'avoir systématiquement de bons résultats.

La lentille optique à vision simultanée progressive pour presbyte suivant l'invention se caractérise ainsi en ce que la courbe représentative de sa proximité P, définie comme étant égale à l'inverse, en dioptries, de la distance D, comptée depuis ladite lentille, à laquelle un rayon lumineux parallèle à son axe et de hauteur $\underline{h}$ par rapport à celui-ci recoupe cet axe, s'inscrit dans une zone comprise entre une courbe enveloppe inférieure $P_{inf}$ et une courbe enveloppe supérieure $P_{sup}$ pouvant être décrites par des polynômes de degré n en h et répondant aux équations suivantes :

$$P_{inf} = f(h) = \left( \Sigma A'_i h^i \right) + P_{VL} \quad (I)$$
$$P_{sup} = f(h) = \left( \Sigma A''_i h^i \right) + P_{VL}$$

dans lesquelles la valeur de $\underline{h}$ est comprise entre 0,4 et 2,2 mm, $P_{VL}$ est la proximité nécessaire pour la vision de loin et $A'_i$, $A''_i$ sont les coefficients des polynômes différents suivant la valeur de l'addition de proximité $\delta_1 = A_{DD}$ correspondant au degré de presbytie du porteur, les valeurs de ces coefficients étant sensiblement les suivantes :

2

pour $A_{DD}$ = 1,5 D :

| | | | | | |
|---|---|---|---|---|---|
| A'0 | = | 12,532267 | A"0 | = | 16,9452 |
| A'1 | = | -92,695892 | A"1 | = | -106,8394 |
| A'2 | = | 305,16919 | A"2 | = | 302,62347 |
| A'3 | = | -513,44922 | A"3 | = | -443,97601 |
| A'4 | = | 476,63852 | A"4 | = | 362,53815 |
| A'5 | = | -247,99097 | A"5 | = | -166,29979 |
| A'6 | = | 67,868942 | A"6 | = | 40,015385 |
| A'7 | = | -7,6131396 | A"7 | = | -3,9203446 |

pour $A_{DD}$ = 2 D :

| | | | | | |
|---|---|---|---|---|---|
| A'0 | = | 23,56555 | A"0 | = | 14,368889 |
| A'1 | = | -182,77804 | A"1 | = | -87,219223 |
| A'2 | = | 605,05684 | A"2 | = | 244,35987 |
| A'3 | = | -1024,1053 | A"3 | = | -337,92626 |
| A'4 | = | 962,99613 | A"4 | = | 241,37509 |
| A'5 | = | -511,24120 | A"5 | = | -85,757212 |
| A'6 | = | 143,7355 | A"6 | = | 12,008102 |

| | | |
|---|---|---|
| A'7 | = | -16,663562 |

pour $A_{DD}$ = 2,5 D :

| | | | | | |
|---|---|---|---|---|---|
| A'0 | = | -28,307575 | A"0 | = | 2,874459 |
| A'1 | = | 190,37743 | A"1 | = | 11,541159 |
| A'2 | = | -445,54529 | A"2 | = | -35,715782 |
| A'3 | = | 512,44763 | A"3 | = | 37,849808 |
| A'4 | = | -315,3125 | A"4 | = | -19,0199096 |
| A'5 | = | 99,678413 | A"5 | = | 4,2867818 |
| A'6 | = | -12,731333 | A"6 | = | -0,28934118 |

pour $A_{DD}$ = 3 D :

| | | | | | |
|---|---|---|---|---|---|
| A'0 | = | 22,19555 | A"0 | = | 57,071102 |
| A'1 | = | -157,74065 | A"1 | = | -357,09277 |
| A'2 | = | 529,74104 | A"2 | = | 1000,8899 |
| A'3 | = | -918,56382 | A"3 | = | -1509,5112 |
| A'4 | = | 881,73279 | A"4 | = | 1311,576 |
| A'5 | = | -475,73774 | A"5 | = | -657,94254 |
| A'6 | = | 135,48897 | A"6 | = | 177,01095 |
| A'7 | = | -15,888513 | A"7 | = | -19,763759 |

et, pour d'éventuelles additions intermédiaires, dont la valeur $\delta$ est comprise entre deux valeurs d'additions $\delta_1$ et $\delta_1$ + 0,5 mentionnées ci-avant, les courbes enveloppes de ces additions intermédiaires sont déduites des courbes enveloppes correspondant à $\delta_1$ et $\delta_1 + 0,5$ par les formules :

$$P^\delta_{inf}(h) = (\frac{\delta - \delta 1}{0,5}) \Delta P_{inf} + P^{\delta 1}_{inf}$$

$$P^\delta_{sup}(h) = (\frac{\delta - \delta 1}{0,5}) \Delta P_{sup} + P^{\delta 1}_{sup}$$

avec

$$\Delta P_{inf} = P^{\delta 1+0,5}_{inf} - P^{\delta 1}_{inf}$$

$$\Delta P_{sup} = P^{\delta 1+0,5}_{sup} - P^{\delta 1}_{sup}$$

L'expérience montre, en effet, que le respect des courbes limites continues, monotones et décroissantes (I) ci-dessus conduit à des résultats très satisfaisants pour le porteur, quel que soit ce porteur, et, notamment, quelle que soit l'addition qui est à lui apporter;

Les caractéristiques et avantages de l'invention ressortiront d'ailleurs de la description qui va suivre, à titre d'exemple, en référence aux dessins schématiques annexés sur lesquels :

la figure 1 est une vue partielle en plan d'une lentille optique suivant l'invention ;

la figure 2 en est une vue partielle en coupe axiale, suivant la ligne II-II de la figure 1 ;

la figure 3A est un diagramme représentatif de la proximité de cette lentille optique pour une première valeur de l'addition ;

les figures 3B 3C, 3D sont des diagrammes analogues à celui de la figure 3A, pour d'autres valeurs de cette addition.

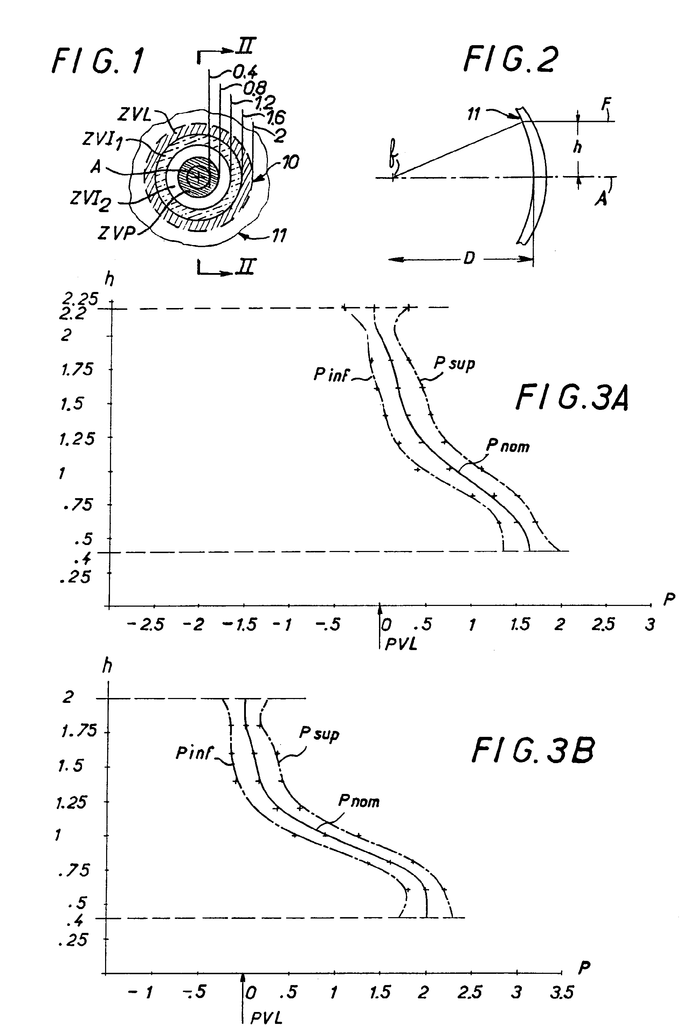

Tel que schématisé sur la figure 1, la zone optique utile 10 de la lentille 11 suivant l'invention se trouve tout entière confinée dans une circonférence de rayon fixé selon l'addition : 2,2 mm pour l'addition 1,5 D et 2 mm pour les additions 2 D, 2,5 D et 3 D. Le rayon de la pupille humaine diminuant, dans les mêmes conditions de luminance, avec l'âge, sa valeur est liée à la valeur d'addition nécessaire au porteur, et est en général pour un sujet presbyte égale ou inférieure à 2,2 mm.

Soit A l'axe de cette lentille 11.

S'agissant, en pratique, dans l'exemple décrit d'une lentille convergente, tout rayon lumineux incident F qui, parallèle à l'axe A, s'étend à une distance $\underline{h}$ de cet axe A, recoupe cet axe A en un point $\underline{f}$ situé à une distance D de la lentille 11 après la traversée de celle-ci.

La proximité P, exprimée en dioptries, est égale à l'inverse de cette distance, exprimée en m.

Si la hauteur $\underline{h}$ est supérieure à 2,2 mm ou 2 mm, les rayons lumineux incidents sont arrêtés par la pupille, et ne concourent pas en pratique à la formation de l'image.

C'est la raison pour laquelle la zone utile 10 de la lentille 11 est limitée, périphériquement, dans l'exemple décrit à une circonférence de rayon égal à 2 mm.

Pour des raisons pratiques, et tel que schématisé en traits interrompus sur la figure 1, cette zone utile 10 est également limitée, au voisinage de l'axe A, par une circonférence de rayon égal à 0,4 mm, étant souligné que la surface de la zone centrale ainsi écartée ne représente qu'une fraction minime, n'excédant pas 4 % de la surface totale de l'ensemble. Dans cette zone centrale, les courbes $P_{inf}(h)$ et $P_{sup}(h)$ s'inscrivent néanmoins dans la continuité des précédentes.

La zone utile 10 comporte, annulairement et concentriquement, tel que schématisé par des hachures sur la figure 1, quatre zones de vision distinctes, à savoir une zone de vision de loin $Z_{VL}$ deux zones de vision intermédiaire $Z_{VI1}$ et $Z_{VI2}$ et une zone de vision de près $Z_{VP}$.

Dans la forme de réalisation représentée, et préférentiellement, les zones $Z_{VL}$, $Z_{VI1}$, $Z_{VI2}$ et $Z_{VP}$ s'étendent de la partie périphérique de la zone utile 10 vers la zone centrale de cette même zone utile.

Mais, si désiré, une disposition inverse peut également être adoptée.

Par simple commodité, et pour des raisons qui apparaîtront ultérieurement, il sera considéré, ci-après, que la zone de vision de près $Z_{VP}$ s'étend entre 0,4 mm et 0,8 mm et que la zone de vision de loin $Z_{VL}$ s'étend entre 1,6 mm et 2 ou 2,2 mm.

Suivant l'invention, la courbe représentative de la proximité P en fonction de la hauteur $\underline{h}$ s'inscrit dans une zone comprise entre une courbe enveloppe inférieure $P_{inf}$ et une courbe enveloppe supérieure $P_{sup}$ répondant aux équations suivantes :

$$P_{inf} = f(h) = (\Sigma A'_i h^i) + P_{VL} \quad (I)$$

$$P_{sup} = f(h) = (\Sigma A''_i h^i) + P_{VL}$$

dans lesquelles $P_{VL}$ est la proximité nécessaire pour la vision de loin et $A'_i$, $A''_i$ sont les coefficients des polynômes différents suivant la valeur de l'addition de proximité $\delta_1 = A_{DD}$ correspondant au degré de presbytie du porteur, les valeurs de ces coefficients étant sensiblement les suivantes :

pour $A_{DD}$ = 1,5 D :

| | | | | |
|---|---|---|---|---|
| A'0 | = | 12,532267 | A"0 = | 16,9452 |
| A'1 | = | -92,695892 | A"1 = | -106,8394 |
| A'2 | = | 305,16919 | A"2 = | 302,62347 |
| A'3 | = | -513,44922 | A"3 = | -443,97601 |
| A'4 | = | 476,63852 | A"4 = | 362,53815 |
| A'5 | = | -247,99097 | A"5 = | -166,29979 |
| A'6 | = | 67,868942 | A"6 = | 40,015385 |
| A'7 | = | -7,6131396 | A"7 = | -3,9203446 |

pour $A_{DD}$ = 2 D :

| | | | | |
|---|---|---|---|---|
| A'0 | = | 23,56555 | A"0 = | 14,368889 |
| A'1 | = | -182,77804 | A"1 = | -87,219223 |
| A'2 | = | 605,05684 | A"2 = | 244,35987 |
| A'3 | = | -1024,1053 | A"3 = | -337,92626 |
| A'4 | = | 962,99613 | A"4 = | 241,37509 |
| A'5 | = | -511,24120 | A"5 = | -85,757212 |
| A'6 | = | 143,7355 | A"6 = | 12,008102 |
| A'7 | = | -16,663562 | | |

pour $A_{DD}$ = 2,5 D :

| | | | | |
|---|---|---|---|---|
| A'0 | = | -28,307575 | A"0 = | 2,874459 |
| A'1 | = | 190,37743 | A"1 = | 11,541159 |
| A'2 | = | -445,54529 | A"2 = | -35,715782 |
| A'3 | = | 512,44763 | A"3 = | 37,849808 |
| A'4 | = | -315,3125 | A"4 = | -19,0199096 |
| A'5 | = | 99,678413 | A"5 = | 4,2867818 |
| A'6 | = | -12,731333 | A"6 = | -0,28934118 |

pour $A_{DD}$ = 3 D :

| | | | | |
|---|---|---|---|---|
| A'0 | = | 22,19555 | A"0 = | 57,071102 |
| A'1 | = | -157,74065 | A"1 = | -357,09277 |
| A'2 | = | 529,74104 | A"2 = | 1000,8899 |
| A'3 | = | -918,56382 | A"3 = | -1509,5112 |
| A'4 | = | 881,73279 | A"4 = | 1311,576 |
| A'5 | = | -475,73774 | A"5 = | -657,94254 |
| A'6 | = | 135,48897 | A"6 = | 177,01095 |
| A'7 | = | -15,888513 | A"7 = | -19,763759 |

et, pour d'éventuelles additions intermédiaires, dont la valeur $\delta$ est comprise entre deux valeurs d'additions $\delta_1$ et $\delta_1$ + 0,5 mentionnées ci-avant, les courbes enveloppes de ces additions intermédiaires sont déduites des courbes enveloppes correspondant à $\delta_1$ et $\delta_1$ + 0,5 par les formules :

$$P_{inf}^{\delta}(h) = (\frac{\delta - \delta 1}{0,5}) \Delta P_{inf} + P_{inf}^{\delta 1}$$

$$P_{sup}^{\delta}(h) = (\frac{\delta - \delta 1}{0,5}) \Delta P_{sup} + P_{sup}^{\delta 1}$$

avec

$$\Delta P_{inf} = P_{inf}^{\delta 1+0,5} - P_{inf}^{\delta 1}$$

$$\Delta P_{sup} = P_{sup}^{\delta 1+0,5} - P_{sup}^{\delta 1}$$

Il s'avère, en effet, que la courbe de proximité d'une lentille ophtalmique constitue une caractéristique de celle-ci, et qu'il est donc possible, en relevant à l'aide de moyens d'analyse appropriés cette courbe de proximité, de procéder à une identification d'une telle lentille optique.

Il s'avère, également, qu'il est possible, partant d'une telle courbe de proximité, de déterminer les surfaces à donner aux faces avant et arrière de cette lentille optique pour qu'elle satisfasse à cette courbe de proximité.

Les dispositions correspondantes relevant de l'homme de l'art, elles ne seront pas décrites ici.

En pratique, la face arrière de la lentille optique 11 suivant l'invention peut par exemple être une surface sphérique, seule la surface de sa face avant étant établie de manière à obtenir la courbe de proximité recherchée.

En pratique également, toute combinaison de surfaces sphérique ou asphérique donnant une courbe de proximité s'inscrivant dans les limites précédemment décrites est envisageable.

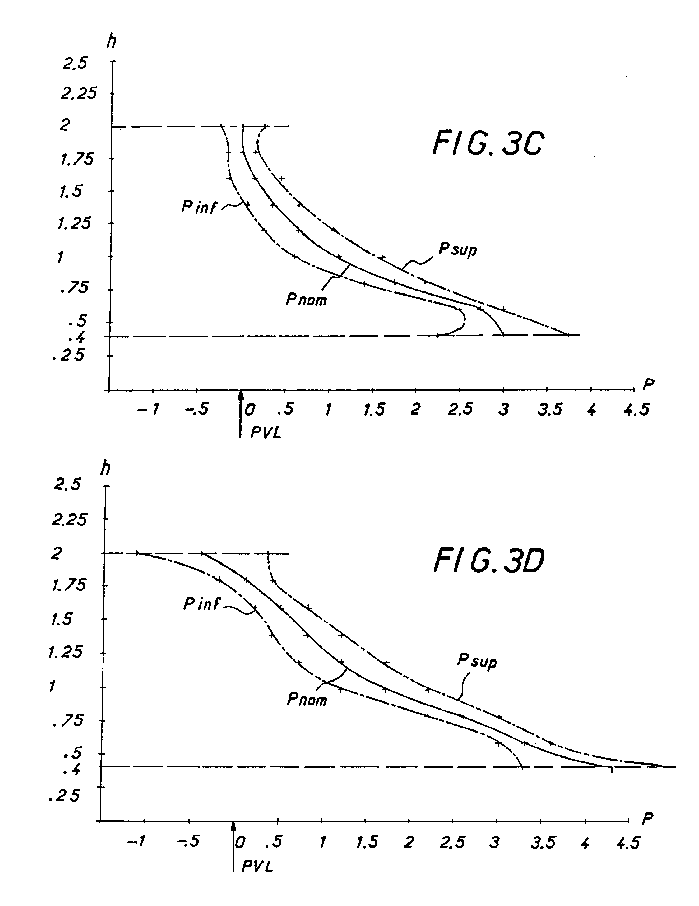

Sur les diagrammes des figures 3A, 3B, 3C et 3D, sur lesquelles ont été portées en abscisses, la proximité P, exprimée en dioptries, et en ordonnées la hauteur $\underline{h}$, exprimée en millimètres, ont été tracées, en traits interrompus, les courbes enveloppes $P_{inf}$, $P_{sup}$ (I) correspondant, respectivement, pour une valeur de proximité en vision de loin $P_{VL}$ égale à 0, à addition de proximité $A_{DD}$ égale à 1,5 pour la figure 3A, 2 pour la figure 3B, 2,5 pour la figure 3C, et 3 pour la figure 3D.

Pour d'autres valeurs de $P_{VL}$, positives ou négatives, les courbes $P_{inf}(h)$ et $P_{sup}(h)$ se déduisent par simple translation.

Sur ces diagrammes ont en outre été tracées, en trait plein, à titre d'exemple, entre les courbes enveloppes $P_{inf}$, $P_{sup}$, une courbe nominale $P_{nom}$ donnant tout particulièrement satisfaction.

Cette courbe nominale $P_{nom}$ répond à l'équation suivante :

$$P_{nom} = f(h) = (\Sigma A_i h^i) + P_{VL}$$

avec des valeurs sensiblement égales aux suivantes pour les coefficients numériques $A_i$ :

pour $A_{DD}$ = 1,5 D :
- A0 = 1,8983333
- A1 = -3,8368794
- A2 = 17,797017
- A3 = -34,095052
- A4 = 28,027344
- A5 = -10,464243
- A6 = 1,4648437
- A7 = 0

pour $A_{DD}$ = 2 D :
- A0 = 12,637321
- A1 = -85,632629
- A2 = 269,61975
- A3 = -425,09732
- A4 = 361,26779
- A5 = -168,43481
- A6 = 40,408779
- A7 = -3,8719125

pour $A_{DD}$ = 2,5 D :
- A0 = -12,716558
- A1 = 100,95929
- A2 = -240,63054
- A3 = 275,14871
- A4 = -167,1658
- A5 = 51,982597
- A6 = -6,5103369

pour $A_{DD}$ = 3 D :

A0 = 39,633326
A1 = -257,41671
A2 = 765,31546
A3 = -1214,0375
A4 = 1096,6544
A5 = -566,84014
A6 = 156,24996
A7 = -17,826136

Ainsi qu'on le notera, les courbes enveloppes inférieures $P_{inf}$ et les courbes enveloppes supérieures $P_{sup}$ suivent globalement, au moins dans leurs partie centrale, la courbe nominale $P_{nom}$ correspondante.

De préférence, le long de l'une des courbes de proximité représentées sur les diagrammes des figures 3A, 3B, 3C et 3D, la valeur locale du gradient de proximité $\dfrac{dP}{dh}$ n'excède pas 5 D/mm de manière continue sur une plage de proximité supérieure à 0,25 D (II).

En outre, comme on peut le voir sur ces figures, la proximité correspondant à la limite inférieure de la zone utile 10 a une valeur supérieure à $(P_{VL} + A_{DD})$.

De préférence, et c'est le cas pour les courbes représentées sur les diagrammes des figures 3A, 3B, 3C, 3D, le gradient moyen de proximité $G_{VP}$ en vision de près, apprécié en fonction des seules coordonnées des points de la courbe nominale $P_{nom}$ correspondant aux limites, précisées ci-dessus, de la zone de vision de près $Z_{VP}$, et le gradient moyen de proximité $G_{VL}$ en vision de loin, apprécié dans les mêmes conditions, sont entre eux dans la relation suivante :

$$\frac{G_{VP}}{G_{VL}} > 2 \quad \text{(III)}$$

De préférence, également, et c'est le cas dans la forme de réalisation représentée, la surface $S_{VP}$ de la section de passage participant utilement à la vision de près, c'est-à-dire, en pratique, la surface de la zone de vision de près $S_{VP}$, et la surface $S_{VL}$ de la section de passage participant utilement à la vision de loin, c'est-à-dire, en pratique, la surface de la zone de vision loin $Z_{VL}$, sont entre elles dans la relation suivante :

$$\frac{S_{VL}}{S_{VP}} \cong 3 \quad \text{(IV)}$$

Il s'avère, en effet, à l'expérience, que ces caractéristiques II, III et IV sont favorables à l'obtention de bons résultats.

Bien entendu, la présente invention ne se limite pas à la forme de réalisation décrite et représentée, mais englobe toute variante d'exécution.

**Revendications**

1.  Lentille optique à vision simultanée pour la correction de la presbytie, caractérisée en ce que la courbe représentative de sa proximité P, définie comme étant égale à l'inverse, en dioptries, de la distance D, comptée depuis ladite lentille, à laquelle un rayon lumineux parallèle à son axe et de hauteur h par rapport à celui-ci recoupe cet axe, s'inscrit dans une zone comprise entre une courbe enveloppe inférieure $P_{inf}$ et une courbe enveloppe supérieure $P_{sup}$ répondant aux équations suivantes :

    $$P_{inf} = f(h) = \left( \Sigma\, A'_i\, h^i \right) + P_{VL}$$
    $$P_{sup} = f(h) = \left( \Sigma\, A''_i\, h^i \right) + P_{VL}$$

    dans lesquelles la valeur h est comprise entre 0,4 et 2,2 mm, $P_{VL}$ est la proximité nécessaire pour la vision de loin et $A'_i$, $A''_i$ sont les coefficients des polynômes différents suivant la valeur de l'addition de proximité $\delta_1 = A_{DD}$ correspondant au degré de presbytie du porteur, les valeurs de ces coefficients étant sensiblement les suivantes :

pour $A_{DD}$ = 1,5 D :

| | | | | | |
|---|---|---|---|---|---|
| A'0 | = | 12,532267 | A"0 | = | 16,9452 |
| A'1 | = | -92,695892 | A"1 | = | -106,8394 |
| A'2 | = | 305,16919 | A"2 | = | 302,62347 |
| A'3 | = | -513,44922 | A"3 | = | -443,97601 |
| A'4 | = | 476,63852 | A"4 | = | 362,53815 |
| A'5 | = | -247,99097 | A"5 | = | -166,29979 |
| A'6 | = | 67,868942 | A"6 | = | 40,015385 |
| A'7 | = | -7,6131396 | A"7 | = | -3,9203446 |

pour $A_{DD}$ = 2 D :

| | | | | | |
|---|---|---|---|---|---|
| A'0 | = | 23,56555 | A"0 | = | 14,368889 |
| A'1 | = | -182,77804 | A"1 | = | -87,219223 |
| A'2 | = | 605,05684 | A"2 | = | 244,35987 |
| A'3 | = | -1024,1053 | A"3 | = | -337,92626 |
| A'4 | = | 962,99613 | A"4 | = | 241,37509 |
| A'5 | = | -511,24120 | A"5 | = | -85,757212 |
| A'6 | = | 143,7355 | A"6 | = | 12,008102 |
| A'7 | = | -16,663562 | | | |

pour $A_{DD}$ = 2,5 D :

| | | | | | |
|---|---|---|---|---|---|
| A'0 | = | -28,307575 | A"0 | = | 2,874459 |
| A'1 | = | 190,37743 | A"1 | = | 11,541159 |
| A'2 | = | -445,54529 | A"2 | = | -35,715782 |
| A'3 | = | 512,44763 | A"3 | = | 37,849808 |
| A'4 | = | -315,3125 | A"4 | = | -19,0199096 |
| A'5 | = | 99,678413 | A"5 | = | 4,2867818 |
| A'6 | = | -12,731333 | A"6 | = | -0,28934118 |

pour $A_{DD}$ = 3 D :

| | | | | | |
|---|---|---|---|---|---|
| A'0 | = | 22,19555 | A"0 | = | 57,071102 |
| A'1 | = | -157,74065 | A"1 | = | -357,09277 |
| A'2 | = | 529,74104 | A"2 | = | 1000,8899 |
| A'3 | = | -918,56382 | A"3 | = | -1509,5112 |
| A'4 | = | 881,73279 | A"4 | = | 1311,576 |
| A'5 | = | -475,73774 | A"5 | = | -657,94254 |
| A'6 | = | 135,48897 | A"6 | = | 177,01095 |
| A'7 | = | -15,888513 | A"7 | = | -19,763759 |

et, pour d'éventuelles additions intermédiaires, dont la valeur δ est comprise entre deux valeurs d'additions δ₁ et δ₁ + 0,5 mentionnées ci-avant, les courbes enveloppes de ces additions intermédiaires sont déduites des courbes enveloppes correspondant à δ₁ et δ₁ + 0,5 par les formules :

EP 0 381 567 B1

$$P_{inf}^{\delta}(h) = (\frac{\delta - \delta 1}{0,5}) \, \Delta \, P_{inf} + P_{inf}^{\delta 1}$$

$$P_{sup}^{\delta}(h) = (\frac{\delta - \delta 1}{0,5}) \, \Delta \, P_{sup} + P_{sup}^{\delta 1}$$

avec

$$\Delta \, P_{inf} = P_{inf}^{\delta 1+0,5} - P_{inf}^{\delta 1}$$
$$\Delta \, P_{sup} = P_{sup}^{\delta 1+0,5} - P_{sup}^{\delta 1}$$

2. Lentille optique suivant la revendication 1, caractérisé en ce que la courbe représentative de sa proximité répond à l'équation suivante

$$P_{nom} = f(h) = (\Sigma \, A_i \, h^i) + P_{VL}$$

avec, dans les mêmes conditions que précédemment,

pour $A_{DD}$ = 1,5 D :

    A0 = 1,8983333
    A1 = -3,8368794
    A2 = 17,797017
    A3 = -34,095052
    A4 = 28,027344
    A5 = -10,464243
    A6 = 1,4648437
    A7 = 0

pour $A_{DD}$ = 2 D :

    A0 = 12,637321
    A1 = -85,632629
    A2 = 269,61975
    A3 = -425,09732
    A4 = 361,26779
    A5 = -168,43481
    A6 = 40,408779
    A7 = -3,8719125

pour $A_{DD}$ = 2,5 D :

    A0 = -12,716558
    A1 = 100,95929
    A2 = -240,63054
    A3 = 275,14871
    A4 = -167,1658
    A5 = 51,982597
    A6 = -6,5103369

pour $A_{DD}$ = 3 D :

    A0 = 39,633326
    A1 = -257,41671
    A2 = 765,31546
    A3 = -1214,0375
    A4 = 1096,6544
    A5 = -566,84014
    A6 = 156,24996
    A7 = -17,826136

3. Lentille optique suivant l'une quelconque des revendications 1, 2, caractérisée en ce que la valeur locale du gradient de proximité $\frac{dP}{dh}$ n'excède pas 5 dioptries par mm de manière continue sur une plage de proximité supérieure à 0,25 dioptrie.

4. Lentille optique suivant l'une quelconque des revendications 1 à 3, caractérisée en ce que le gradient moyen de proximité, $G_{VP}$ en vision de près et le gradient moyen de proximité $G_{VL}$ en vision de loin sont entre eux dans la relation suivante :

$$\frac{G_{VP}}{G_{VL}} > 2$$

9

5. Lentille ophtalmique suivant l'une quelconque des revendications 1 à 4, caractérisée en ce que la surface $S_{VP}$ de la section de passage participant utilement à la vision de près de la surface $S_{VL}$ de la section de passage participant utilement à la vision de loin sont entre elles dans la relation suivante :

$$\frac{S_{VL}}{S_{VP}} \geqq 3$$

**Patentansprüche**

1. Optische Linse mit gleichzeitiger Sicht zur Korrektur der Presbyopie, dadurch gekennzeichnet, daß die repräsentative Kurve ihrer Nähe P, die in Dioptrien gleich dem Kehrwert des Abstands D, der von der Linse gerechnet wird, definiert ist, in dem ein zu ihrer Achse paralleler Lichtstrahl mit der Höhe $\underline{h}$ bezüglich dieser diese Achse schneidet, in einer Zone zwischen einer unteren Mantelkurfe $P_{inf}$ und einer oberen Mantel-kurve $P_{sup}$ gelagert ist, die den folgenden Gleichungen genügen:

$$P_{inf} = f(h) = ( \Sigma A'_i\, h^i ) + P_{VL} \quad (I)$$
$$P_{sup} = f(h) = ( \Sigma A''_i\, h^i ) + P_{VL}$$

in denen der Wert von $\underline{h}$ zwischen 0,4 und 2,2 mm liegt und $P_{VL}$ die für die Weitsicht notwendige Nähe ist und $A'_i$, $A''_i$ die Koeffizienten der unterschiedlichen Polynome gemäß dem Wert der Addition der Nähe $\delta_1 = A_{DD}$ entsprechend dem Grad der Presbyopie des Trägers sind, wobei die Werte dieser Koeffizienten praktisch die folgenden sind:

```
für A_DD    =    1,5 D :
    A'0    =    12,532267           A''0    =    16,9452
    A'1    =   -92,695892           A''1    =  -106,8394
    A'2    =   305,16919            A''2    =   302,62347
    A'3    =  -513,44922            A''3    =  -443,97601
    A'4    =   476,63852            A''4    =   362,53815
    A'5    =  -247,99097            A''5    =  -166,29979
    A'6    =    67,868942           A''6    =    40,015385
    A'7    =   - 7,6131396          A''7    =   - 3,9203446


für A_DD    =    2 D:
    A'0    =    23,56555            A''0    =    14,368889
    A'1    =  -182,77804            A''1    =   -87,219223
    A'2    =   605,05684            A''2    =   244,35987
    A'3    =  -1024,1052            A''3    =  -337,92626
    A'4    =   962,99613            A''4    =   241,37509
    A'5    =  -511,24120            A''5    =   -85,757212
    A'6    =   143,7355             A''6    =    12,008102
    A'7    =  -16,663562
```

für $A_{DD}$ = 2,5 D:

| | | | | | |
|---|---|---|---|---|---|
| A'0 | = | -28,307575 | A''0 | = | 2,874459 |
| A'1 | = | 190,37743 | A''1 | = | 11,541159 |
| A'2 | = | -445,54529 | A''2 | = | -35,715782 |
| A'3 | = | 512,44763 | A''3 | = | 37,849808 |
| A'4 | = | -315,3125 | A''4 | = | -19,0199096 |
| A'5 | = | 99,678413 | A''5 | = | 4,2867818 |
| A'6 | = | -12,731333 | A''6 | = | -0,28934118 |

für $A_{DD}$ = 3 D:

| | | | | | |
|---|---|---|---|---|---|
| A'0 | = | 22,19555 | A''0 | = | 57,071102 |
| A'1 | = | -157,74065 | A''1 | = | -357,09277 |
| A'2 | = | 529,74104 | A''2 | = | 1000,8899 |
| A'3 | = | -918,56382 | A''3 | = | -1509,5112 |
| A'4 | = | 881,73279 | A''4 | = | 1311,576 |
| A'5 | = | -475,73774 | A''5 | = | -657,94254 |
| A'6 | = | 135,48897 | A''6 | = | 177,01095 |
| A'7 | = | -15,888513 | A''7 | = | -19,763759 |

und für eventuelle Zwischenadditionen, deren Wert zwischen zwei vorher genannten Additionswerten $\delta_1$ und $\delta_1 + 0,5$ liegt, werden die Mantelkurven dieser Zwischenadditionen von den Mantelkurven entsprechend $\delta_1$ und $\delta_1 + 0,5$ durch die Formeln abgeleitet :

$$P_{inf}^{\delta}(h) = (\frac{\delta - \delta 1}{0,5}) \Delta P_{inf} + P_{inf}^{\delta 1}$$

$$P_{sup}^{\delta}(h) = (\frac{\delta - \delta 1}{0,5}) \Delta P_{sup} + P_{sup}^{\delta 1}$$

mit

$$\Delta P_{inf} = P_{inf}^{\delta 1+0,5} - P_{inf}^{\delta 1}$$

$$\Delta P_{sup} = P_{sup}^{\delta 1+0,5} - P_{sup}^{\delta 1}$$

2. Optische Linse nach Anspruch 1, dadurch gekennzeichnet, daß die repräsentative Kurve ihrer Nähe der folgenden Gleichung genügt:

$$P_{nom} = f(h) = (\Sigma A_i h^i) + P_{VL}$$

mit, unter den gleichen Bedingungen wie vorher :

für $A_{DD}$ = 1,5 D

A0 = 1,8983333
A1 = -3,8368794
A2 = 17,797017
A3 = -34,095052
A4 = 28,027344
A5 = -10,464243
A6 = 1,4648437
A7 = 0

für $A_{DD}$ = 2 D:

A0 = 12,637321
A1 = -85,632629
A2 = 269,61975
A3 = -425,09732

A4 = 361,26779
A5 = -168,43481
A6 = 40,408779
A7 = -3,8719125
für $A_{DD}$ = 2,5 D:
A0 = -12,716558
A1 = 100,95929
A2 = -240,63054
A3 = 275,14871
A4 = -167,1658
A5 = 51,982597
A6 = -6,5103369
für $A_{DD}$ = 3 D:
A0 = 39,633326
A1 = -257,41671
A2 = 765,31546
A3 = -1214,0375
A4 = 1096,6544
A5 = -566,84014
A6 = 156,24996
A7 = -17,826136

3. Optische Linse nach irgendeinem der Ansprüche 1, 2, dadurch gekennzeichnet, daß der örtliche Wert des Nähe-Gradienten $\frac{dP}{dh}$ nicht 5 Dioptrie pro mm fortlaufend über einen höheren Nähe-Bereich als 0,25 D überschreitet.

4. Optische Linse nach irgendeinem der Ansprüche 1 bis 3, dadurch gekennzeichnet, daß der mittlere Gradient der Nähe $G_{VP}$ in Nahsicht und der mittlere Gradient der Nähe $G_{VL}$ in Weitsicht zueinander in der folgenden Relation stehen :

$$\frac{G_{VP}}{G_{VL}} > 2$$

5. Ophtalmische Linse nach irgendeinem der Ansprüche 1 bis 4, dadurch gekennzeichnet, daß die Fläche $S_{VP}$ des Durchgangsabschnittes, der an der Nahsicht mitwirkt und die Fläche $S_{VL}$ des Durchgangsabschnittes, der an der Weitsicht mitwirkt, zueinander in der folgenden Relation stehen :

$$\frac{S_{VL}}{S_{VP}} \geqq 3$$

## Claims

1. A multifocal optical lens for the correction of presbyopia, characterised in that the curve which is representative of its proximity P which is defined as being equal to the inverse in dioptres of the distance D, reckoned from said lens, at which a light ray parallel to its axis and of a height h with respect to same intersects said axis, is inscribed in a zone between a lower envelope curve Pinf and an upper envelope curve $P_{sup}$ complying with the following equations:

$$P_{inf} = f(h) = (\Sigma A'_i h^i) + P_{VL}$$
$$P_{sup} = f(h) = (\Sigma A''_i h^i) + P_{VL}$$

in which the value of h is between 0.4 and 2.2 mm, $P_{VL}$ is the proximity necessary for far vision and $A'_i$, $A''_i$ are the coefficients of the polynomials which differ according to the value of the addition of proximity $\delta_1 = A_{DD}$ corresponding to the degree of presbyopia of the wearer, the values of said coefficients being substantially as follows:

```
for A_DD    =   1.5 D :
    A'O     =     12.532267        A"O    =    16.9452
    A'1     =    -92.695892        A"1    =  -106.8394
    A'2     =    305.16919         A"2    =   302.62347
    A'3     =   -513.44922         A"3    =  -443.97601
    A'4     =    476.63852         A"4    =   362.53815
    A'5     =   -247.99097         A"5    =  -166.29979
    A'6     =     67.868942        A"6    =    40.015385
    A'7     =     -7.6131396       A"7    =    -3.9203446
for A_DD    =   2 D :
    A'O     =     23.56555         A"O    =    14.368889
    A'1     =   -182.77804         A"1    =   -87.219223


    A'2     =    605.05684         A"2    =   244.35987
    A'3     =  -1024.1053          A"3    =  -337.92626
    A'4     =    962.99613         A"4        241.37509
    A'5     =   -511.24120         A"5    =   -85.757212
    A'6     =    143.7355          A"6    =    12.008102
    A'7     =    -16.663562
for A_DD    =   2.5 D :
    A'O     =    -28.307575        A"O    =     2.874459
    A'1     =    190.37743         A"1    =    11.541159
    A'2     =   -445.54529         A"2    =   -35.715782
    A'3     =    512.44763         A"3    =    37.849808
    A'4     =   -315.3125          A"4    =   -19.0199096
    A'5     =     99.678413        A"5    =     4.2867818
    A'6     =    -12.731333        A"6    =    -0.28934118
for A_DD    =   3 D :
    A'O     =     22.19555         A"O    =    57.071102
    A'1     =   -157.74065         A"1    =  -357.09277
    A'2     =    529.74104         A"2    =  1000.8899
    A'3     =   -918.56382         A"3    = -1509.5112
    A'4     =    881.73279         A"4    =  1311.576
    A'5     =   -475.73774         A"5    =  -657.94254
    A'6     =    135.48897         A"6    =   177.01095
    A'7     =    -15.888513        A"7    =   -19.763759
```

and, for possible intermediate additions whose value $\delta$ is between two values of additions $\delta_1$ and $\delta_1 + 0.5$ mentioned hereinbefore, the envelope curves of said intermediate additions are deduced from the envelope curves corresponding to $\delta_1$ and $\delta_1 + 0.5$ by the following formulae:

$$P_{inf}^{\delta}(h) = \left(\frac{\delta - \delta 1}{0.5}\right) \Delta P_{inf} + P_{inf}^{\delta 1}$$

$$P_{sup}^{\delta}(h) = \left(\frac{\delta - \delta 1}{0.5}\right) \Delta P_{sup} + P_{sup}^{\delta 1}$$

with

$$\Delta P_{inf} = P_{inf}^{\delta 1+0.5} - P_{inf}^{\delta 1}$$
$$\Delta P_{sup} = P_{sup}^{\delta 1+0.5} - P_{sup}^{\delta 1}$$

2. An optical lens according to claim 1 characterised in that the curve which is representative of its proximity complies with the following equation:

$$P_{nom} = f(h) = (\Sigma A_i h^i) + P_{VL}$$

with, under the same conditions as before,

for $A_{DD} = 1.5$ D :

    A0 = 1.8983333
    A1 = -3.8368794
    A2 = 17.797017
    A3 = -34.095052
    A4 = 28.027344
    A5 = -10.464243
    A6 = 1.4648437
    A7 = 0

for $A_{DD} = 2$ D :

    A0 = 12.637321
    A1 = -85.632629
    A2 = 269.61975
    A3 = -425.09732
    A4 = 361.26779
    A5 = -168.43481
    A6 = 40.408779
    A7 = -3.8719125

for $A_{DD} = 2.5$ D :

    A0 = -12.716558
    A1 = 100.95929
    A2 = -240.63054
    A3 = 275.14871
    A4 = -167.1658
    A5 = 51.982597
    A6 = -6.5103369

for $A_{DD} = 3$ D :

    A0 = 39.633326
    A1 = -257.41671
    A2 = 765.31546
    A3 = -1214.0375
    A4 = 1096.6544
    A5 = -566.84014
    A6 = 156.24996
    A7 = -17.826136

3. An optical lens according to either one of claims 1 and 2 characterised in that the local value of the gradient of proximity $\frac{dP}{dh}$ does not exceed 5 dioptres per mm continuously over a range of proximity of greater than 0.25 dioptre.

4. An optical lens according to any one of claims 1 to 3 characterised in that the mean gradient of proximity $G_{VP}$ in near vision and the mean gradient of proximity $G_{VL}$ in far vision are related to each other in the following manner:

$$\frac{G_{VP}}{G_{VL}} > 2$$

5. An ophthalmic lens according to any one of claims 1 to 4 characterised in that the surface area $S_{VP}$ of the passage section usefully participating in near vision and the surface area $S_{VL}$ of the passage section usefully participating in far vision are related to each other in the following manner:

$$\frac{S_{VL}}{S_{VP}} \geqq 3$$

FIG.1

FIG.2

FIG.3A

FIG.3B

FIG.3C

FIG.3D